# EUROPEAN PATENT APPLICATION

(11) **EP 3 398 650 A1**
(43) Date of publication of application: **07.11.2018**
(21) Application number: 17169084.5
(22) Date of filing: 02.05.2017
(51) Int. Cl.: A61N 1/375

(54) **BIOCOMPATIBLE COMPOSITE SYSTEM**

(71) Applicant: Heraeus Deutschland GmbH & Co. KG, 63450 Hanau (DE); Heraeus Quartz America LLC, Austin, TX 78728 (US)
(72) Inventor: Donelon, Matthew Joseph, Phoenix, AZ 85048 (US); Dittmer, Robert, 63450 Hanau (DE); Hausch, Ulrich, 60318 Frankfurt am Main (DE); Trötzschel, Jens, 63549 Ronneburg (DE)
(74) Representative: Heraeus IP

(57) **Abstract**

The invention relates to a biocompatible composite system, an implantable medical device, and a manufacturing method for a biocompatible composite system. The biocompatible composite system comprises a printed multi-layer circuit board and a feedthrough. The circuit board comprises at least one electrically conductive via. The feedthrough comprises an insulating ceramic base material and at least one electrically conductive cermet pathway. The pathway is embedded into the base material to form an electrically conductive and hermetically sealed connection to the via of the circuit board. The feedthrough and the circuit board are integrated into one composite part.

## Description

### FIELD OF THE INVENTION

The invention relates to a biocompatible composite system, an implantable medical device, and a manufacturing method for a biocompatible composite system.

### BACKGROUND OF THE INVENTION

An implantable medical device may be understood as a device which is set up to perform a medical function and which can be introduced into body tissue. An active implantable medical device may be understood as an implantable medical device that can conduct electrical signals from a hermetically sealed housing to a part of the body tissue of the user and/or receive electrical signals from the part of the body tissue of the user.

Currently, active implantable medical devices comprise the following parts:
a) An internal electronic component, which is typically mounted to a printed circuit board, represents an active part of the active implantable medical device, and comprises, e.g., a microprocessor, a battery, capacitors, etc.
b) A housing, typically made from Ti or a Ti alloy, to house the internal electronic component and protect it from body fluids.
c) A feedthrough, typically made from pins brazed into a ceramic, integrated into a ferrule that is attached to the housing enabling full hermetic sealing and, at the same time, allowing for feeding electrical signals in and out.
d) A header, typically made from metal connectors that are connected to the feedthrough and overmolded into silicone to connect a lead to the active implantable medical device.

However, such construction provides at least one of the following disadvantages:
- A high single part count due to the complex and differential construction approach,
- a complex supply chain due to the high single part count,
- a high number of junctions/connections that require machines, time, and staff for manufacture, and
- consequently, an increase of total costs.
- Further, a high number of potential failure modes due to the high single part count and the number of connections,
- which decreases the reliability of the system, and
- a limited potential for miniaturization again due to the complex construction.

### SUMMARY OF THE INVENTION

Hence, there may be a need to provide an improved biocompatible composite system, which in particular allows reducing the manufacturing costs.

The problem of the present invention is solved by the subject matters of the independent claims, wherein further embodiments are incorporated in the dependent claims. It should be noted that the aspects of the invention described in the following apply also to the biocompatible composite system, the implantable medical device, and the manufacturing method for a biocompatible composite system.

According to the present invention, a biocompatible composite system is presented. The biocompatible composite system comprises a printed multi-layer circuit board and at least a feedthrough.

The circuit board comprises at least one electrically conductive via.

The feedthrough comprises an insulating ceramic base material and at least one electrically conductive cermet pathway.

The pathway is embedded into the base material to form an electrically conductive and hermetically sealed connection to the via of the circuit board.

The feedthrough and the circuit board are integrated into one composite part which may be in one or multiple planes.

The term "cermet" can be understood as a composite material made of one or more ceramic materials in at least one metallic matrix or a composite material made of one or more metallic materials in at least one ceramic matrix. The term "pathway" can be understood as electrically conducting element which generally means an element set up to establish an electrical connection between at least two sites and/or at least two elements. The term "hermetically sealed" can be understood in that moisture and/or gases cannot permeate through a hermetically sealed element at all or only to a minimal extent upon the intended use. The term "integrated" can be understood here in that feedthrough and circuit board form only one body, they cannot be two separate bodies. More detailed and other definitions can be found at the end of this section.

The biocompatible composite system with integrated feedthrough and circuit board may be or may be used as an (active) implantable medical device and also for any other kind of encapsulated device.

The ceramic base material and the cermet pathway of the feedthrough form a cermet-ceramic composite material system that allows the integration of conductive pathways into a hermetic, biocompatible, integrated composite system. As a result, the circuit board and the feedthrough can be integrated into one part, which greatly simplifies the overall assembly complexity and cost.

In other words, one advantage of the present composite system is that a conventional separate feedthrough is avoided by the integrated feedthrough. Thereby, wire connections inside the device and a need for mounting techniques to secure the feedthrough inside the device are also avoided.

Consequently, the integrated feedthrough according to the invention leads to a much more robust device. This is very advantageous, because durability of the device, e.g., in form of a pacemaker is often critical. Increasing the life span of the device is, therefore, very valuable to the manufacturer.

Further advantages of the present composite system are a decreased single part count, drastic simplification of the assembly, less manual labor in assembly, increased robustness and reliability, better miniaturization potential through increased degree of integration, increased comfort and safety for the patient, and significantly reduced total costs.

The circuit board may be a cuboid with two base surfaces and four lateral surfaces, wherein the base surfaces are larger than the lateral surfaces. In an example, the circuit board comprises at least one electric contact arranged at a lower or upper base surface of the circuit board. A length and a width of the feedthrough is smaller than a length and a width of one of the base surfaces of the circuit board. In an example, the feedthrough comprises at least one electric contact arranged at a lateral surface, an end face or at a base surface of the feedthrough outside the circuit board. The number of electric contacts at the circuit board and at the feedthrough can range from one to as many as desired which will fit and perform electrically.

The via of the circuit board may comprise metal traces or may be made of a cermet. In the latter case, the via of the circuit board, the ceramic base material and the cermet pathway of the feedthrough may form a cermet-ceramic composite material system that allows the integration of vias and pathways into one hermetic, biocompatible, integrated composite system. This can be used to further reduce the overall assembly complexity and its costs.

One option to design the present composite system is to combine the circuit board with a feedthrough in the shape of a tab, which extends past a normal area of the circuit board. In other words, the feedthrough is a tab protruding from a lateral surface of the circuit board. The tab may be a cuboid with smaller base surfaces and/or lateral surfaces than the circuit board. The tab may also be a cylinder and in particular a flattened cylinder with smaller diameter and/or height than the lateral surfaces and/or base surfaces of the circuit board. The tab can have electric contacts located on one, two or all three edges of the tab depending upon the desired connection type. The electric contacts may also be located on the top or bottom surface of the tab.

In an example, the feedthrough and in particular the tab lies in a different plane than the circuit board. In other words, the plane of the feedthrough is offset from the plane of the circuit board. This allows providing more room for surface mount components as, e.g., batteries or coils.

Another option to design the present composite system is to combine the circuit board with a feedthrough in form of a protruding section. In other words, the feedthrough is a tower protruding from a base surface of the circuit board. The tower may be a cuboid with smaller base surfaces and/or lateral surfaces than the circuit board. The tower may also be a cylinder with smaller diameter and/or height than the base surfaces and/or lateral surfaces of the circuit board. This layout allows placing a header at a lateral side of the circuit board. The header is explained further below.

Another option to design the present composite system is to combine the circuit board with a feedthrough integrated into a side of the circuit board. In other words, the feedthrough is part of a lateral surface of the circuit board.

Another option to design the present composite system is a planar design that incorporates circuit board and feedthrough in one body, namely in the body of the circuit board. In other words, the feedthrough is part of a base surface of the circuit board. The composite system may also incorporate circuit board, feedthrough and the header as explained in the following. In other words, the biocompatible composite system further comprises the header for connecting the feedthrough to a lead of a medical device, wherein also the header is integrated into the composite part. The header may comprise one or more leads. As a result, not only the circuit board and the feedthrough are integrated into one part, but also the header, which further simplifies the overall assembly complexity and cost. For example, a surface mount/through wire circuit board, a header and a feedthrough in one component can be used in a pacemaker and other implantable medical devices. Such composite can also be used for any other electronic device that requires a hermetically sealed environment.

In an example, the biocompatible composite system further comprises a housing surrounding the circuit board at least partially. The housing may also comprise a ferrule. In an example, the biocompatible composite system further comprises a ferrule connected with the circuit board and provided with an opening surrounding the feedthrough. The ferrule may be a washer shaped disc with an opening sized to match the feedthrough. This ferrule can be made of the material of the housing or other compatible material. This allows the ferrule to be brazed to the circuit board before the components are solder attached. This brazing may take place at temperatures higher than the surface mount components are able to survive. The ferrule can also be one of the halves or main pieces of the housing. After the components are attached, the last weld of the halves or pieces of the housing can occur. This last weld may be a laser weld, which needs to occur in a manner, which does not damage any internal components. If for some reason it is needed to have a feedthrough at each end of a device, this technique can be done from more than one location on the circuit board such as having, e.g., two tabs for the feedthrough.

The circuit board may have any design needed. The circuit board can be constructed with as many layers as necessary. Other layers may be included which are, e.g., only for increasing thickness and thereby strengthening of the circuit board. The circuit board can be made of biocompatible ceramic materials typically including alumina and various platinum or other noble metals or corrosion resistant metals.

The circuit board may be a multi-layer circuit made, e.g., by a planar approach using a HTCC (high temperature co-fired ceramics) or LTCC (low temperature co-fired ceramics) process. This planar approach may be formed so that it occupies one or more planes due to the plastic nature of the composite before sintering. When using HTCC or LTCC, components are usually made in multiples in one sheet and cut apart in a green (unfired) state or can be cut after sintering to full density. HTCC and LTCC essentially guarantees a passing of thermal cycling testing required for circuit boards. Further, they allow very strong devices from an electrical isolation standpoint, which allows a use of high stress currents to proof test circuit paths.

Contact pad areas may be located on one or both sides of the circuit board for the attachment of electrical components. The electrical components can be surface mounted to either side of the circuit board that can accommodate a surface mounted component. To make electric contacts, e.g., in form of edge contact pads, it may be necessary to print an electric contact on a side edge after the part has been cut. The edge of the part can be made with a radius or chamfer by laser ablating the surfaces in a region where a cut will take place. This laser ablation can be a "trench" which may have a variety of shapes, which can be beneficial in replacement of a sharp right angle corner. Circuit paths can be printed on individual tape layers that have paths printed one or both sides. The tape layers may have vias to allow a circuit path to pass between layers.

Pastes used to create components of the present composite system can be chosen especially to have hermetic properties to meet helium leak requirements for devices that need particular levels of hermetic properties.

In an example, the circuit board/feedthrough composite part comprises at least one of a group of resistors, inductors and capacitors incorporated into the composite part by HTCC or LTCC. In particular, the circuit board may have the capability of incorporating resistors, inductors and capacitors within the printed circuit inside or on outer layers of the circuit board. Having resistors on the outer surface allows a laser trimming to correct a resistor to a specific resistance. According to the present invention, also an implantable medical device is presented. The implantable medical device comprises the composite system as described above and an electric and/or electronic device arranged at least partially in electrical contact with the composite system.

According to the present invention, also a manufacturing method for a biocompatible composite system is presented. It comprises the following steps:
a) forming a multi-layer circuit board and a feedthrough, and
b) sintering the multi-layer circuit board and the feedthrough.

The circuit board comprises at least one electrically conductive via. The feedthrough comprises an insulating ceramic base material and at least one electrically conductive cermet pathway. The pathway is embedded into the base material to form an electrically conductive and hermetically sealed connection to the via of the circuit board. The feedthrough and the circuit board are integrated into one composite part.

The term "integrated" can be understood in that feedthrough and circuit board are formed as only one body and in particular simultaneously. There is no separate and/or subsequent forming of feedthrough and circuit board.

In an example, the manufacturing method is a High Temperature Cofired Ceramics (HTCC) method in which a number of layers are generated and assembled into the composite system. In an example, the forming step therefore comprises a forming of a layer, which is repeated to form a stack of layers, which form the composite system.

In another example, the manufacturing method is an additive manufacturing method. In an example, the additive manufacturing method comprises one of a group of printing, 3D printing, ceramic injection molding, co-extrusion, powder pressing and/or the like to form the composite system.

In an example, the sintering is a co-sintering or co-firing. In other words, the term "sintering" may only be a process of densification or may also include a process of removing the binder ("debinding") in the meaning of "firing". In an example, the manufacturing method further comprises a step of isostatic pressing.

In an example, the manufacturing method for a biocompatible composite system further comprises a machining of the layers before and/or after the sintering or co-firing. The machining may be done by laser or mechanically, e.g., by drilling, milling, grinding etc. In an example, the providing comprises a providing of feedthrough material and of expendable material. The expendable material may be understood as sacrificial material. In an example, the sintering or co-firing comprises a removing of the expendable material so that the feedthrough material remains. In detail:
The circuit board may be manufactured in a way that connections are established by means of internal cermet pathways. The feedthrough, e.g., in form of a protruding tower may provide means to attach the, e.g., titanium ferrule by, e.g., gold brazing. The feedthrough tower may be manufactured by machining or by use of the expendable material for the surrounding, whereas the expendable material vanishes during sintering or co-firing and only the non-expendable cermet and ceramic remain, forming the tower with its internal pathways. The expendable material can be carbon black powder or other polymer powders, which take up space yet burn away during a binder burn out process. The expendable material allows a uniform compaction/consolidation of the layers during pressing of the layers into one body.

In an example, the manufacturing method further comprises a laser ablating to form a radius or chamfer at an edge. This laser ablation can be a "trench" which may have a variety of shapes, which can be beneficial in replacement of a sharp right angle corner.

In an example, the manufacturing method further comprises a laser trimming of a resistor of the circuit board, which allows correcting a resistor to a specific resistance.

In the following, definitions of terms are provided.

The term implant encompasses an (active) implantable medical device and shall include any device which is set up to perform at least one medical function and which can be introduced into a body tissue of a human or animal user. As a general rule, the medical function can include any function selected from the group consisting of a therapeutic function, a diagnostic function, and a surgical function. For example, the medical function can include at least one actuator function, in which an actuator is used to exert at least one stimulus on the body tissue, for example, an electrical stimulus.

All implants can usually include, for example, at least one housing, for example, at least one hermetically sealed housing. The housing can in one embodiment enclose at least one electronics unit, for example, a triggering and/or analytical electronics unit of the implant.

The term active implantable medical device shall include all implantable medical devices that can conduct electrical signals from a hermetically sealed housing to a part of the body tissue of the user and/or receive electrical signals from the part of the body tissue of the user. Accordingly, the term, active implantable medical device, includes, for example, cardiac pacemakers, cochlea implants, implantable cardioverters/defibrillators, nerve, brain, organ or muscle stimulators as well as implantable monitoring devices, hearing aids, retinal implants, muscle stimulators, implantable drug pumps, artificial hearts, bone growth stimulators, prostate implants, stomach implants or the like.

A housing of an implant shall be understood to be an element that encloses, at least in part, at least one functional element of the implantable medical device that is set up to perform the at least one medical function or promotes the medical function. For example, the housing includes at least one internal space that takes up the functional element fully or in part. For example, the housing can be set up to provide mechanical protection to the functional element from stresses occurring during operation and/or handling, and/or protection to the functional element from influences of its surroundings such as, for example, influences of a body fluid. The housing can, for example, border and/or close the implant with respect to the outside.

The housing may be provided to be hermetically sealed such that, for example, the internal space is hermetically sealed with respect to the external space. In this context, the term, "hermetically sealed", can illustrate that moisture and/or gases cannot permeate through the hermetically sealed element at all or only to a minimal extent upon the intended use for the common periods of time (for example 5-10 years). The so-called leak rate, which can be determined, for example, by leak tests, is a physical parameter that can describe, for example, a permeation of gases and/or moisture through a device, for example, through the electrical bushing and/or the housing. Pertinent leak tests can be carried out with helium leak testers and/or mass spectrometers and are specified in the Mil-STD-883G Method 1014 standard. In this context, the maximal permissible helium leak rate is determined as a function of the internal volume of the device to be tested. According to the methods specified in MIL-STD-883G, method 1014, section 3.1 and taking into consideration the volumes and cavities of the devices to be tested that are used in the application of one embodiment, said maximal permissible helium leak rates can, for example, be from 1x10-8 atm*cm3/sec to 1x10-7 atm*cm3/sec.

A pathway shall be understood as a conducting element or electrically conducting element, which generally means an element set up to establish an electrical connection between at least two sites and/or at least two components. For example, the conducting element can include one or more electrical conductors, for example metallic conductors. In the scope of one embodiment, the conducting element is made fully or partly of at least one cermet pathway.

The term hermetically sealed shall be understood, for example, to mean that the device to be tested (for example the housing and/or the electrical bushing and/or the housing with the electrical bushing) has a helium leak rate of less than 1x10-7 atm*cm3/sec. In one embodiment, the helium leak rate can be less than 1x10-8 atm*cm3/sec, in one embodiment, less than 1x10-9 atm*cm3/sec. For the purpose of standardization, the above-mentioned helium leak rates can also be converted into the equivalent standard air leak rate. The definition of the equivalent standard air leak rate and the conversion are specified in the ISO 3530 standard.

For example, a pathway may be hermetically sealed with respect to the insulator, at least in part. In this context, hermetic sealing shall be understood to mean a sealing, which in one embodiment is designed to be hermetically sealed, in one embodiment as such and without any contribution of further elements, whereby reference shall be made to the definition provided above with regard to the term, "hermetically sealed". However, in addition, the term, "hermetically sealed, at least in part" can include refinements, in which the pathway is hermetically sealed with respect to the electrically insulating insulator involving a contribution of one or more further element(s).

The term insulating or electrically insulating shall be understood to mean a resistivity of at least 107 Ohm*m, in one embodiment, of at least 108 Ohm*m, in one embodiment of at least 109 Ohm*m, and in one embodiment of at least 1011 Ohm*m. For example, an insulating material can be provided such that, as mentioned above, a flow of current between a conducting element and the housing and/or between multiple conducting elements is prevented, at least largely, for example through the resistivity values between the conducting element and the housing. For example, the insulating material can comprise at least one ceramic material. The insulating material may comprise additions of magnesium oxide (MgO), silicon oxide (SiO2) or glass. The insulating material can, for example, be made fully or partly from one or more sinterable materials, for example, from one or more ceramic-based sinterable materials.

The term cermet shall refer to a composite material made of one or more ceramic materials in at least one metallic matrix or a composite material made of one or more metallic materials in at least one ceramic matrix. For production of a cermet, for example, a mixture of at least one ceramic powder and at least one metallic powder can be used to which, for example, at least one binding agent and, if applicable, at least one solvent can be added. The ceramic powder or powders of the cermet in one embodiment have a mean grain size of less than 10 µm, in one embodiment less than 5 µm, and in one embodiment less than 3 µm. The metallic powder or powders of the cermet in one embodiment have a mean grain size of less than 15 µm, in one embodiment less than 10 µm, and in one embodiment less than 5 µm. For production of an insulator, for example, at least one ceramic powder can be used to which, for example, at least one binding agent and, if applicable, at least one solvent can be added. In this context, the ceramic powder or powders in one embodiment has/have a mean grain size of less than 10 µm (1 µm is equal to 1x10-6 m), in one embodiment less than 5 µm, in one embodiment less than 3 µm. For example, the median value or the d50 value of the grain size distribution is considered to be the mean grain size in this context. The d50 value corresponds to the value at which 50 percent of the grains of the ceramic powder and/or metallic powder are finer and 50 percent are coarser than the d50 value.

A ceramic manufacturing method shall be understood to mean a procedure that includes at least one sintering process of at least one insulating and/or at least one cermet material. As shall be explained in more detail below, said ceramic manufacturing method can, for example, include a forming for the manufacture of at least one form body, for example one ceramic green compact and/or at least one ceramic brown compact.

A sintering or a sintering process shall generally be understood to mean a procedure for the manufacture of materials or work-pieces, in which powdered, for example, fine-grained, ceramic and/or metallic substances are heated and connected in the process. This process can proceed without applying external pressure onto the substance to be heated or can, for example, proceed under elevated pressure onto the substance to be heated, for example under a pressure of at least 2 bar, in one embodiment higher pressures, for example pressures of at least 10 bar, for example, at least 100 bar, or even at least 1000 bar. The process can proceed, for example, fully or partly, at temperatures below the melting temperature of the powdered materials, for example at temperatures of 700 degrees centigrade to 1700 degrees centigrade. The process can be carried out, for example, fully or partly, in a tool and/or a mold such that a forming step can be associated with the sintering process. Aside from the powdered materials, a starting material for the sintering process can include further materials, for example one or more binding agents and/or one or more solvents. The sintering process can proceed in one or more steps, whereby additional steps can precede the sintering process, for example one or more forming steps and/or one or more debinding steps.

The sintering process, for example, of the cermet, but of the insulating material just as well, for example, can proceed comparable to a sintering process that is commonly used for homogeneous powders. For example, the material can be densified in the sintering process at high temperature and, if applicable, high pressure such that the cermet is virtually sealed tight or has no more than closed porosity.

For the pathway according to one embodiment, the at least one ceramic component of the cermet can include, for example, at least one of the following materials: glass in general, aluminum oxide (Al2O3), magnesium oxide (MgO), silicon oxide (SiO2), zirconium dioxide (ZrO2), zirconium oxide-toughened aluminum oxide (ZTA-Zirconia Toughened Aluminum-Al2O3/ZrO2), yttrium-toughened zirconium oxide (Y-TZP), aluminum nitride (AIN), magnesium oxide (MgO), piezoceramic materials, barium(Zr, Ti) oxide, barium(CE, Ti) oxide, and sodium-potassium-niobate.

For the pathway according to one embodiment, the at least one metallic component of the cermet can include, for example, at least one of the following metals and/or an alloy based on at least one of the following metals: platinum, iridium, niobium, molybdenum, tantalum, a tantalum alloy, tungsten, a tungsten alloy, titanium, cobalt, chromium, zirconium.

An electrically conductive connection is usually established in the cermet pathway when the metal content exceeds the so-called percolation threshold at which the metal particles in the sintered cermet are connected to each other, at least in spots, such that electrical conduction is enabled. For this purpose, experience tells that the metal content should be 25 percent by volume and more, in one embodiment 32 percent by volume, in one embodiment more than 38 percent by volume, depending on which materials have been selected. The metal content can, for example, be 25 percent by volume to 90 percent by volume, in one embodiment 32 percent by volume to 60 percent by volume, and in one embodiment 38 percent by volume to 50 percent by volume. The electrically conductive connection may also depend on a constitution of the material, as, e.g., grain size, grain shape, and grain distribution.

In the scope of one embodiment, the terms, "including a cermet," "comprising a cermet," and "cermet-containing", are used synonymously. Accordingly, the terms refer to the property of an element, being that the element contains cermet. This meaning also includes the variant of an embodiment in that the element, for example the pathway element, consists of a cermet, i.e., is fully made of a cermet.

Finally, the term electric contact as used herein shall be understood as an electrical element which may be active or passive, i.e., which may supply electrical current in the form of pulses or continuously to, for example, a tissue in a human or animal body, or which may be suitable to measure a parameter like a sensor, wherein the parameter may, for example, be a temperature, a pressure, a movement, a chemical value like a pH-value or a presence of a specific chemical component.

It shall be understood that the biocompatible composite system, the implantable medical device, and the manufacturing method for a biocompatible composite system according to the independent claims have similar and/or identical preferred embodiments, in particular, as defined in the dependent claims. It shall be understood further that a preferred embodiment of the invention can also be any combination of the dependent claims with the respective independent claim.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the accompanying drawings:
Figure 1 shows a schematic drawing of an example of a biocompatible composite system.
Figures 2a to 2c show schematic drawings of another example of a biocompatible composite system.
Figure 3 shows a schematic drawing of another example of a biocompatible composite system.
Figures 4a and 4b show schematic drawings of another example of a biocompatible composite system.
Figure 5 shows a schematic drawing of an example of an implantable medical device 1.
Figure 6 shows a schematic overview of steps of a manufacturing method for a biocompatible composite system.

### DETAILED DESCRIPTION OF EMBODIMENTS

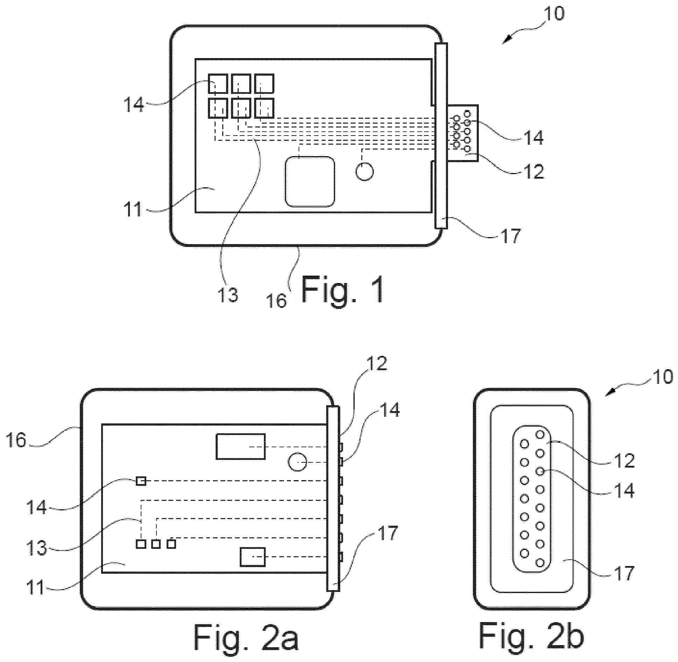

Figure 1 shows a schematic top view of an example of a biocompatible composite system 10 according to the invention. The biocompatible composite system 10 may be used for active implantable medical devices.

The biocompatible composite system 10 comprises a printed multi-layer circuit board 11 and a feedthrough 12. The circuit board 11 is a cuboid (with rounded edges) with two base surfaces and four lateral surfaces, wherein the base surfaces are larger than the lateral surfaces. A length and a width of the feedthrough 12 is smaller than a length and a width of one of the base surfaces of the circuit board 11. The circuit board 11 comprises several electrically conductive cermet vias 13 and several electric contacts 14 in form of contact pads arranged at an upper surface of the circuit board 11.

The feedthrough 12 comprises an insulating ceramic base material and at least one electrically conductive cermet pathway. The insulating ceramic base material may comprise Al2O3 with high purity and some minor additions like magnesium oxide (MgO) or silicon oxide (SiO2). Cermet is a composite material made of one or more ceramic materials in at least one metallic matrix or a composite material made of one or more metallic materials in at least one ceramic matrix.

The pathway is a conducting element that can include one or more electrical conductors. It is embedded into the base material to form an electrically conductive and hermetically sealed connection to the via 13 of the circuit board 11. Hermetically sealed means that moisture and/or gases cannot permeate through the hermetically sealed element at all or only to a minimal extent.

The feedthrough 12 and the circuit board 11 are integrated into one composite part which means that feedthrough 12 and circuit board 11 form one body.

The feedthrough 12 comprises several electric contacts 14 in form of contact pads arranged at an upper surface of the feedthrough 12 outside the circuit board 11.

The biocompatible composite system 10 further comprises an, e.g., metal housing 16 surrounding the circuit board 11 and a ferrule 17 connected with the circuit board 11 and providing an opening surrounding the feedthrough 12. The ferrule 17 is a washer shaped disc with an opening sized to match the feedthrough 12.

Figure 1 shows one option to design the present composite system 10, namely a feedthrough 12 in the shape of a tab, which extends past a normal area of the circuit board 11. In other words, the feedthrough 12 is a tab protruding from a lateral surface of the circuit board 11. The tab is a cuboid with smaller base surfaces and lateral surfaces than the circuit board 11.

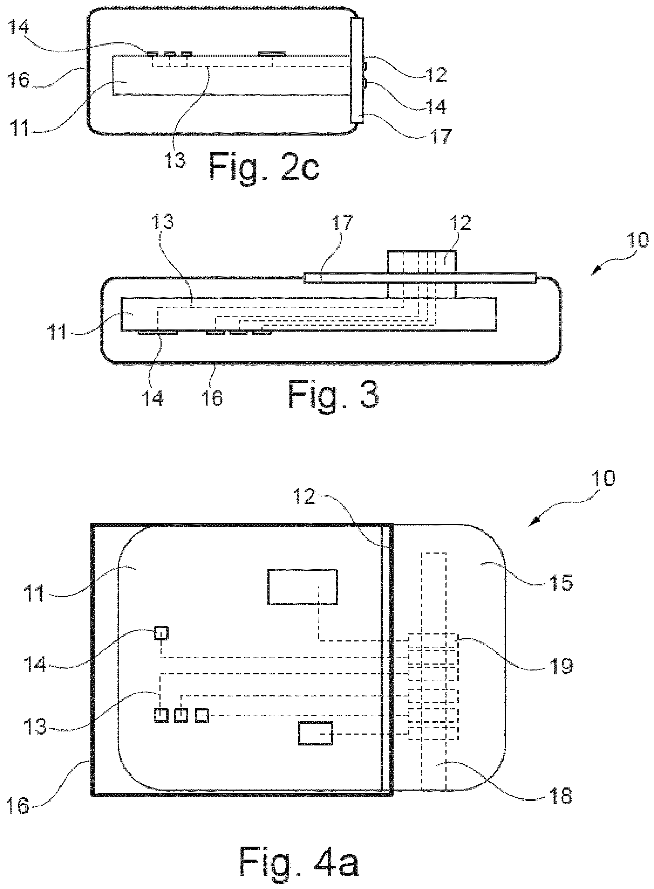

Figures 2a to 2c show a schematic top view (Figure 2a) and two side views (Figures 2b and 2c) of another example of a biocompatible composite system 10. They show another option to design the present composite system 10, namely to combine the circuit board 11 with a feedthrough 12 integrated into a side of the circuit board 11. Integrated into the side of the circuit board 11 means that the feedthrough 12 is part of the lateral surface of the circuit board 11 and not separate or distinct to the circuit board 11. All other elements correspond to the ones shown in Figure 1.

Figure 3 shows a schematic drawing of another example of a biocompatible composite system 10. Figure 3 shows another option to design the present composite system 10, namely to combine the circuit board 11 with a feedthrough 12 in form of a protruding section. In other words, the feedthrough 12 is a tower protruding from a base surface of the circuit board 11. The tower is a cuboid with smaller base surfaces and lateral surfaces than the circuit board 11. All other elements correspond to the ones shown in Figure 1.

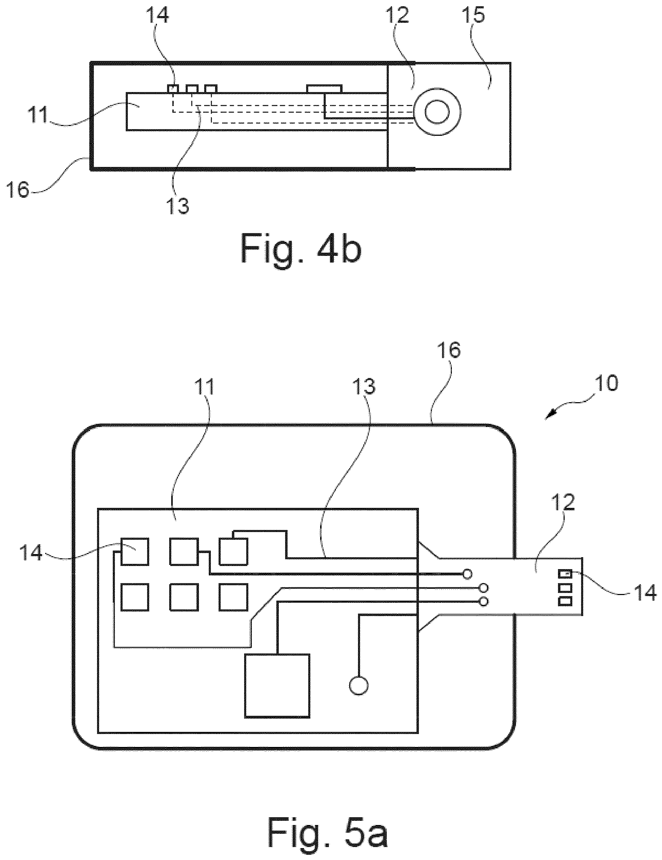

Figures 4a and 4b show a schematic top view (Figure 2a) and a side view (Figure 2b) of another example of a biocompatible composite system 10. They show another option to design the present composite system 10, namely a planar design that incorporates even more components, namely the circuit board 11, the feedthrough 12 and a header 15. The feedthrough 12 is part of a base surface of the circuit board 11. The header 15 serves for connecting the feedthrough 12 to a lead of a medical device (not shown). The header 15 therefore comprises a lead inlet 18 and internal connections rings 19 for connecting the lead to the composite system 10. All other elements correspond to the ones shown in Figure 1. As a result, not only the circuit board 11 and the feedthrough 12 are integrated into one part, but also the header 15, which further simplifies the overall assembly complexity and cost. For example, such surface mount/through wire circuit board 11, the header 15 and the feedthrough 12 in one component can be used in a pacemaker and other implantable medical devices.

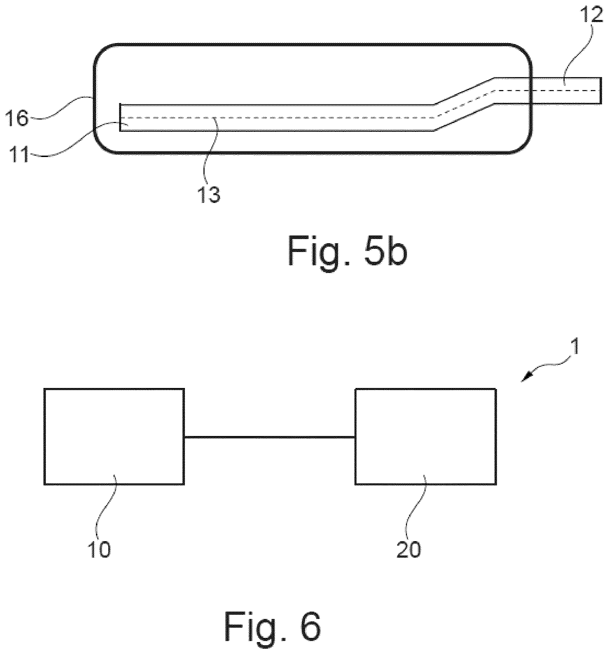

Figures 5a and 5b show an example of the type of Figure 1 where the feedthrough 12 and in particular the tab lies in a different plane than the circuit board 11. In other words, the plane of the feedthrough 12 is offset from the plane of the circuit board 11.

Figure 6 shows a schematic drawing of an example of an implantable medical device 1 according to the invention. The implantable medical device 1 comprises the composite system 10 as described above and an electronic device 20 arranged in electrical contact with the composite system 10.

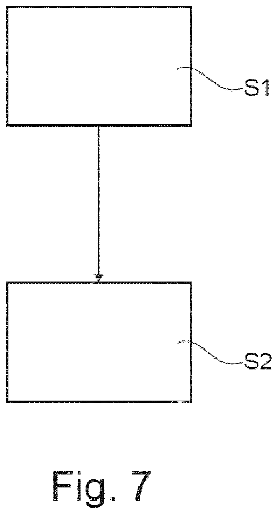

Figure 7 shows a schematic overview of steps of a manufacturing method for a biocompatible composite system 10 according to the invention. The method comprises the following steps, not necessarily in this order:
- In a first step S1, forming a multi-layer circuit board 11 and a feedthrough 12.
- In a second step S2, sintering the multi-layer circuit board 11 and the feedthrough 12.

The circuit board 11 comprises at least one electrically conductive via 13. The feedthrough 12 comprises an insulating ceramic base material and at least one electrically conductive cermet pathway. The pathway is embedded into the base material to form an electrically conductive and hermetically sealed connection to the via 13 of the circuit board 11. The feedthrough 12 and the circuit board 11 are integrated into one composite part.

The term "integrated" means that feedthrough 12 and circuit board 11 are simultaneously formed as only one body. The forming step may be done by means of a High Temperature Cofired Ceramics (HTCC) method or an additive manufacturing method as, e.g., 3D printing.

The providing step S1 may comprise a providing of feedthrough material and of expendable material. The expendable material may be understood as sacrificial material as, e.g., carbon black powder or a polymer powder. The sintering then comprises a removing of the expendable material so that the feedthrough 12 material remains.

The manufacturing method may further comprise a step of machining the layers before and/or after the sintering by laser or mechanically drilling, milling, grinding etc. The manufacturing method may further comprise a step of laser ablating to form a radius or chamfer at an edge. The manufacturing method may further comprise a step of laser trimming of a resistor of the circuit board 11.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A biocompatible composite system (10), comprising:
- a printed multi-layer circuit board (11), and
- a feedthrough (12),
wherein the circuit board (11) comprises at least one electrically conductive via (13), wherein the feedthrough (12) comprises an insulating ceramic base material and at least one electrically conductive cermet pathway, wherein the pathway is embedded into the base material to form an electrically conductive and hermetically sealed connection to the via (13) of the circuit board (11), and wherein the feedthrough (12) and the circuit board (11) are integrated into one composite part.

2. Composite system (10) according to claim 1, wherein the feedthrough (12) is a tab protruding from a lateral surface of the circuit board (11).

3. Composite system (10) according to claim 1, wherein the feedthrough (12) is a tower protruding from a base surface of the circuit board (11).

4. Composite system (10) according to claim 1, wherein the feedthrough (12) is part of a lateral surface of the circuit board (11).

5. Composite system (10) according to claim 1, wherein the feedthrough (12) is part of a base surface of the circuit board (11).

6. Composite system (10) according to one of the preceding claims, wherein the plane of the feedthrough (12) is offset from the plane of the circuit board (11).

7. Composite system (10) according to one of the preceding claims, wherein the feedthrough (12) comprises at least one electric contact (14) arranged at a lateral surface, an end face or at a base surface of the feedthrough (12) outside the circuit board (11).

8. Composite system (10) according to one of the preceding claims, further comprising a header (15) for connecting the feedthrough (12) to a lead of a medical device, wherein also the header (15) is integrated into the composite part.

9. Composite system (10) according to one of the preceding claims, further comprising a housing (16) surrounding the circuit board (11) at least partially.

10. Composite system (10) according to one of the preceding claims, further comprising a ferrule (17) connected with the circuit board (11) and provided with an opening surrounding the feedthrough (12).

11. Composite system (10) according to one of the preceding claims, wherein the circuit board/feedthrough composite part comprises at least one of a group of resistors, inductors and capacitors incorporated into the composite part by HTCC or LTCC.

12. Composite system (10) according to one of the preceding claims, wherein the composite system (10) is an implantable medical device.

13. An implantable medical device (1), comprising:
- the composite system (10) according to one of the preceding claims, and
- an electric and/or electronic device (20) arranged at least partially in electrical contact with the composite system (10).

14. A manufacturing method for a biocompatible composite system (10), comprising the steps of:
- forming a multi-layer circuit board (11) and a feedthrough (12), and
- sintering the multi-layer circuit board (11) and the feedthrough (12),
wherein the circuit board (11) comprises at least one electrically conductive via (13), wherein the feedthrough (12) comprises an insulating ceramic base material and at least one electrically conductive cermet pathway, wherein the pathway is embedded into the base material to form an electrically conductive and hermetically sealed connection to the via (13) of the circuit board (11), and wherein the feedthrough (12) and the circuit board (11) are integrated into one composite part.

15. Manufacturing method according to the preceding claim, further comprising a machining of the layers before and/or after the sintering.

16. Manufacturing method according to the preceding claim, further comprising a laser ablation to form a radius or chamfer at an edge.

17. Manufacturing method according to one of the preceding claims, further comprising a laser trimming of a resistor of the circuit board (11).

18. Manufacturing method according to one of the preceding claims, wherein the providing comprises a providing of feedthrough material and of expendable material, and wherein the sintering comprises a removing of the expendable material so that the feedthrough material remains.
